# EUROPEAN PATENT APPLICATION

(11) **EP 3 120 866 A1**
(43) Date of publication of application: **25.01.2017**
(21) Application number: 15178209.1
(22) Date of filing: 24.07.2015
(51) Int. Cl.: A61K 38/48, A01N 63/02, A01P 1/00

(54) **USE OF MARINE SERINE PROTEASES FOR REMOVAL, PREVENTION AND INHIBITION OF FORMATION AND GROWTH OF BIOFILMS**

(71) Applicant: Zymetech ehf., 101 Reykjavik (IS)
(72) Inventor: GUDMUNDSDOTTIR, Agusta, 101 Reykjavik (IS); STEFANSSON, Bjarki, 101 Reykjavik (IS); SCHEVING, Reynir, 101 Reykjavik (IS)
(74) Representative: Awapatent AB

(57) **Abstract**

The present invention relates to the use of marine serine proteases for removal, prevention and inhibition of formation and growth of biofilms.

## Description

### Field of the Invention

The present invention relates to the use of marine serine proteases for removal, prevention and inhibition of formation and growth of biofilms.

### Background of the invention

Biofilms are heterogeneous, complex 3D matrices that comprise a population of microbial cells, which are embedded in an extracellular matrix (ECM). They are not just passive assemblages of cells, but are structurally and dynamically complex biological systems that form local ecosystems. The microbial cells within a biofilm population appear to co-operate and take on special functions. By co-operating and forming a protective ECM the biofilm provides the microorganisms with a protected mode of growth that allows them to colonise diverse environments. The biofilm mode of growth allows the bacteria to counter the immune system of the host as well as antibiotics and similar bacteriostatic and bactericidal agents. The development of biofilm thus allows for a bacteria population to show resistance to antibiotics, and bacteria growing in biofilms are more difficult to defeat than their planktonic, i.e. free-living, counterparts.

The microbial cells of biofilms comprise mono or poly-bacterial populations well known to adhere to biological or non-biological surfaces or interfaces. In waste water treatment systems, non adhering microbial flocs represent a less studied but important alternative biofilm lifestyle. In said multicellular populations, cells adhere to each other. Bacterial lifestyles are regulated by several systems that are closely tied to environmental and nutritional cues. A majority of bacterial species, as well as archaea, protozoa, fungi and algae, have the ability to adhere to surfaces and to each other and to form biofilm structures.

Formation of a biofilm typically begins with the attachment of free-floating microorganisms to a surface. When the expressions of numerous genes are changed, a planktonic cell undergoes a phenotypic shift and switches from the free living mode to the biofilm mode of growth. The first colonists adhere to the surface initially through weak, reversible adhesion, which may become stronger by production of cell adhesion structures such as pili. Once colonization has begun, the biofilm grows through a combination of cell division and appearance and binding of new bacteria. The first colonists facilitate the arrival of other cells by providing more diverse adhesion sites and by beginning to build the matrix that holds the biofilm together.

Biofilms may form in a variety of settings, such as in the nature, domestic industrial and hospital settings, where they exert various affects to which depending on the context, may be positive or negative.

In the medical setting, biofilms form persistent reservoirs of bacteria on surfaces. Biofilms can occur both directly on a patient and indirectly on surfaces of the patient's immediate environment. Biofilms present directly on a patient is commonly associated with recurrent infections, while the transfer of bacteria from the immediate environment to the patient is implicated in both primary and recurrent infections. Examples of biofilm in hospital settings are biofilms on catheters and other forms of tubing, and on implants such as heart valves and joint prosthesis.

In the industrial settings biofilms can both be essential and detrimental. For example, in recent developments of efficient microbial bioreactors, biofilm colonised electrodes are used to generate electricity. Biofilms have also been explored as possible biological factories of compounds, e.g. cellulose.

International patent application WO0078332 provides use of fish serine proteases including trypsins and chymotrypsin derived from cod such as Atlantic cod for treating and/or preventing a variety of diseases and disorders such as inflammatory diseases, infectious diseases caused by viruses, bacteria and fungal species and diseases where a receptor binding mechanism is involved in the pathogenesis.

Trypsin from cod has been shown to facilitate removal of dead skin by debridement and thereby aids in the normal skin repair process. The main problem of using hydrophilic marine enzymes, like cod trypsin and other serine proteases from cold environments, is that such enzymes are sensitive to inactivation by heat and are therefore relatively unstable at room temperature (Stefansson B. et al. 2010 Comparative Biochemistry and Physiology B - Bichemistry and Molecular Biology 155(2) 186-194). The use of cod trypsin in cosmetics, medical devices and pharmaceuticals is dependent on increasing the stability of the enzyme.

The objects of the present invention include provision of means for prevention and inhibition of formation and growth of biofilms.

### Description of the invention

The inventors have identified that marine serine proteases are useful for removal, prevention and inhibition of formation and growth of biofilms.

### Brief description of the drawings

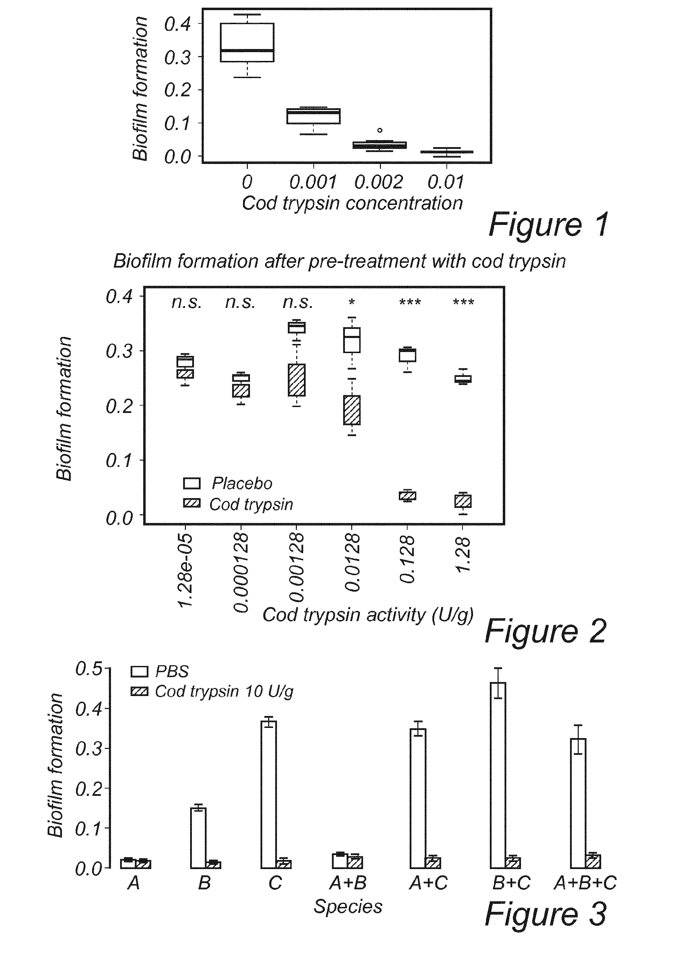

Figure 1. Effects of cod trypsins pre-treatment on biofilm formation. The figure shows that cod trypsins prevent biofilm formation in a concentration dependent manner. Biofilms using a combination of *Streptococcus pneumonia* and *Streptococcus mitis* were grown in a microtiter plate after being treated with cod trypsins or placebo. After allowing the biofilms to grow for 4 hours the wells were stained with crystal violet followed by dissolution with acetic acid. Biofilm formation was measured as absorbance at 492nm, and normalized to untreated biofilm. Based on the study it can be concluded that cod trypsins are very effective in preventing bacterial biofilm formation. Data is presented as a boxplot where the top of the rectangle indicates the third quartile, a horizontal line near the middle of the rectangle indicates the median, and the bottom of the rectangle indicates the first quartile. A vertical line extends from the top of the rectangle to indicate the maximum value, and another vertical line extends from the bottom of the rectangle to indicate the minimum value.
Figure 2. Effects of cod trypsin pre-treatment on biofilm formation. The figure shows that cod trypsins prevent biofilm formation in a concentration dependent manner. Biofilms using a combination of *Streptococcus pneumonia* and *Streptococcus mitis* were grown in a microtiter plate after being treated with cod trypsins or placebo. After allowing the biofilms to grow for 4 hours the wells were stained with crystal violet followed by dissolution with acetic acid. Biofilm formation was measured as absorbance at 492nm, and normalized to untreated biofilm. Based on the study it can be concluded that cod trypsins are very effective in preventing bacterial biofilm formation. Data is presented as a boxplot where the top of the rectangle indicates the third quartile, a horizontal line near the middle of the rectangle indicates the median, and the bottom of the rectangle indicates the first quartile. A vertical line extends from the top of the rectangle to indicate the maximum value, and another vertical line extends from the bottom of the rectangle to indicate the minimum value. Data for placebo and cod trypsin treated bacteria are placed on the same graph. Overlap of the boxes would indicate no difference in biofilm formation. Statistical significance of the difference, as evaluated by Student's t test, is indicated by symbols above the boxes where n.s. is p > 0.05, * is p < 0.05, ** p < 0.01, and *** p < 0.001.
Figure 3. Efficacy of cod trypsin on biofilms formed by super biofilm bacterial species alone and combined. The figure shows the response of the biofilms, formed by the three bacterial species (A, B, C) found in the super biofilm either alone or in combination with each other (A+B, A+C, B+C, A+B+C), to cod trypsins (10 U/g) or PBS treatment. Species: A = *Streptococcus pneumonia,* B = *Streptococcus mitis,* C = *Streptococcus mitis.*

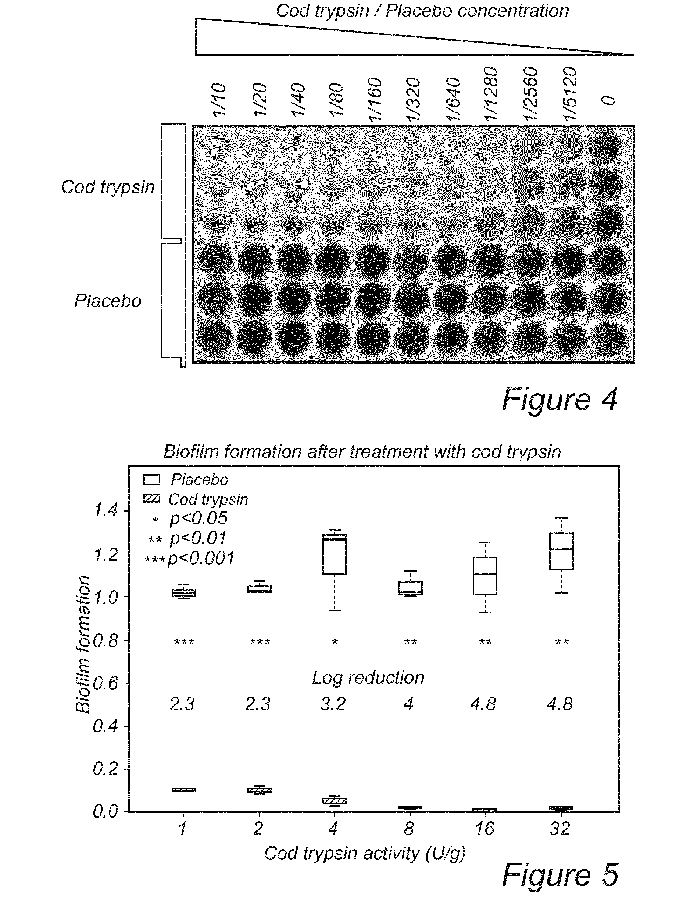

Figure 4. Effects of cod trypsins on the super biofilm presented visually after biofilm staining with crystal violet. The figure shows that cod trypsins disrupt the super biofilm in a concentration dependent manner as seen by the clear wells indicating absence of biofilm. The super biofilm with a combination of *Streptococcus pneumonia* and *Streptococcus mitis* were grown in a microtiter plate. The biofilm was treated with cod trypsins or placebo for 2 minutes and the wells were stained to measure the presence (black wells) of biofilm or its absence (clear wells) after treatment. Based on the study it can be concluded that cod trypsins are very effective in disrupting the super biofilm.
Figure 5. Effects of cod trypsins on the super biofilm as measured by spectrophotometry after crystal violet staining and dissolution in acetic acid. The figure shows that cod trypsins disrupt the super biofilm in a concentration dependent manner. Biofilms using a combination of *Streptococcus pneumonia* and *Streptococcus mitis* were grown in a microtiter plate. The biofilm was treated with cod trypsins or placebo and the wells were stained with crystal violet followed by dissolution with acetic acid. Biofilm formation was measured as absorbance at 492nm, and normalized to untreated biofilm. Based on the study it can be concluded that cod trypsins are very effective in disrupting bacterial biofilm. Data is presented as a boxplot where the top of the rectangle indicates the third quartile, a horizontal line near the middle of the rectangle indicates the median, and the bottom of the rectangle indicates the first quartile. A vertical line extends from the top of the rectangle to indicate the maximum value, and another vertical line extends from the bottom of the rectangle to indicate the minimum value. Data for placebo and cod trypsins treated bacterial biofilms are placed on the same graph. Overlap of the boxes would indicate no difference in biofilm formation, while greater distance between the boxes in the same column indicates a greater difference as represented by the Log reduction of biofilm formation.

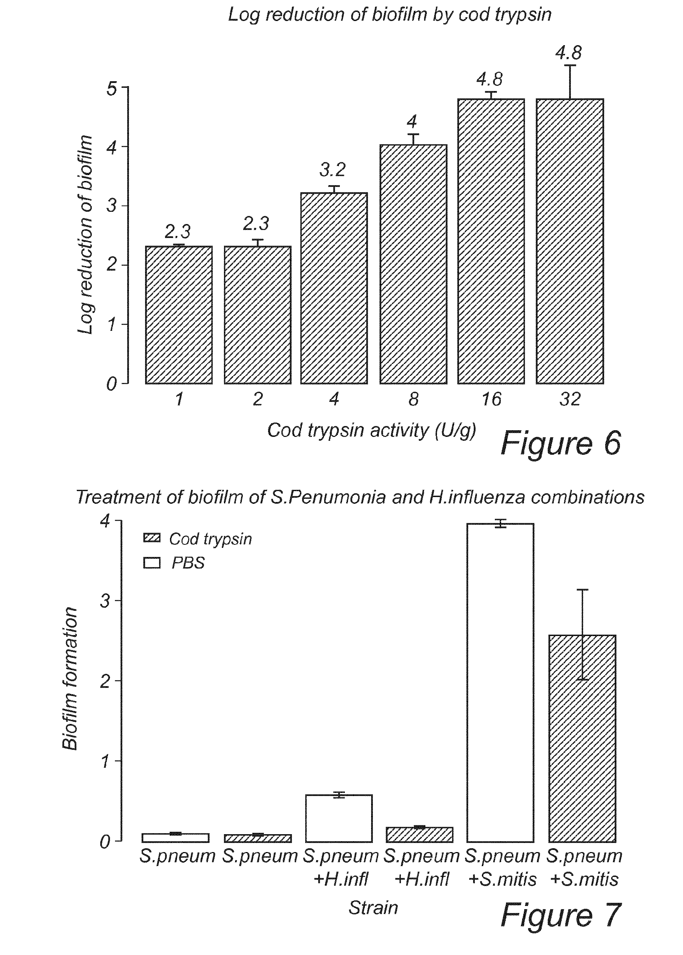

Figure 6. Log reduction of super biofilm by cod trypsins at different concentrations. The figure shows that cod trypsins disrupt the super biofilm in a concentration dependent manner. Biofilms using a combination of *Streptococcus pneumonia* and *Streptococcus mitis* were grown in a microtiter plate. The biofilm was treated with cod trypsins or placebo and the wells were stained with crystal violet followed by dissolution with acetic acid. Biofilm formation was measured as absorbance at 492nm, and the reduction compared to placebo treated biofilm on a logarithmic scale. Based on the study it can be concluded that cod trypsins are very effective in disrupting bacterial biofilm at concentrations 4 U/g or higher, in less than 2 minutes, where a log reduction of 3 represent 99.9% removal of the biofilm.
Figure 7. Treatment of biofilms formed by *S.penumonia* and *H.influenza* or *S.mitis* combinations with cod trypsins. The figure shows that biofilms formed by the super biofilm species *(S. pneumonia* and the two *S.mitis* strains, columns 5 and 6) are far stronger than *S.pneumonia alone* (columns 1 and 2) or *S.pneumonia* in combination with *H.influenza* (column 3 and 4). The super biofilm is also more tolerant to PBS (white columns) and low concentration cod trypsins (black columns) washing. Biofilms using a combination of *Streptococcus pneumonia* and *Streptococcus mitis* or *Haemophilus influenza* were grown in a microtiter plate. The biofilm was washed briefly with cod trypsins or PBS and the wells were stained with crystal violet followed by dissolution with acetic acid. Biofilm formation was measured as absorbance at 492nm, and normalized to untreated biofilm.

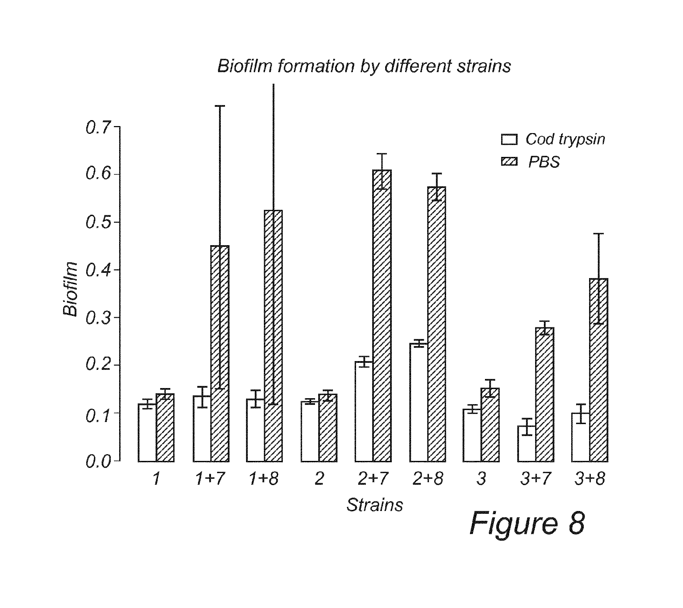

Figure 8. Cod trypsins (white columns) are effective in disrupting biofilms formed by combinations of three strains of *S.pneumonia* and two strains of *H*. *Influenza.* Strains 1, 2, and 3 are *S*. *pneumonia species,* and strains 7 and 8 are *H. Influenza* species. The graph shows that cod trypsins are effective at degrading biofilms of all combinations of all bacterial strains. Biofilms using a combination of *Streptococcus pneumonia* and/or *Haemophilus influenza* were grown in a microtiter plate. The biofilm was treated with cod trypsins or PBS and the wells were stained with crystal violet followed by dissolution with acetic acid. Biofilm formation was measured as absorbance at 492nm. It is clear from the figure that single species biofilms are weak and can easily be disrupted by washing with PBS. On the other hand the stronger biofilms formed by two or more bacterial species require cod trypsin treatment for disruption.

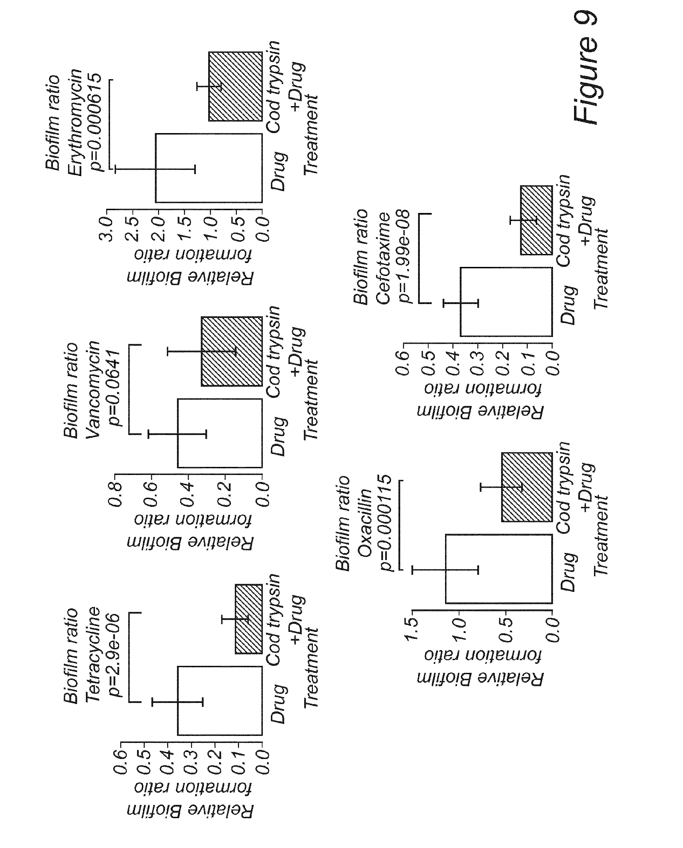

Figure 9. Effects of combination of cod trypsins with selected antibiotics on biofilm dispersal. The figure shows that the combination of cod trypsins and selected antibiotics can be more effective at disrupting biofilms than either alone. Biofilms using a combination of *Streptococcus pneumonia* and *Streptococcus mitis* were grown in a microtiter plate. The biofilm was treated with an antibiotic or a combination of the antibiotics and cod trypsins. The biofilms were stained with crystal violet followed by dissolution with acetic acid. Biofilm formation was measured as absorbance at 492nm, and normalized to untreated biofilm. Based on the study it can be concluded that cod trypsin and antibiotic combinations are more effective in disrupting bacterial biofilm than antibiotics alone. P values above bars indicate significance of difference between the two treatments as evaluated by Student's t test.

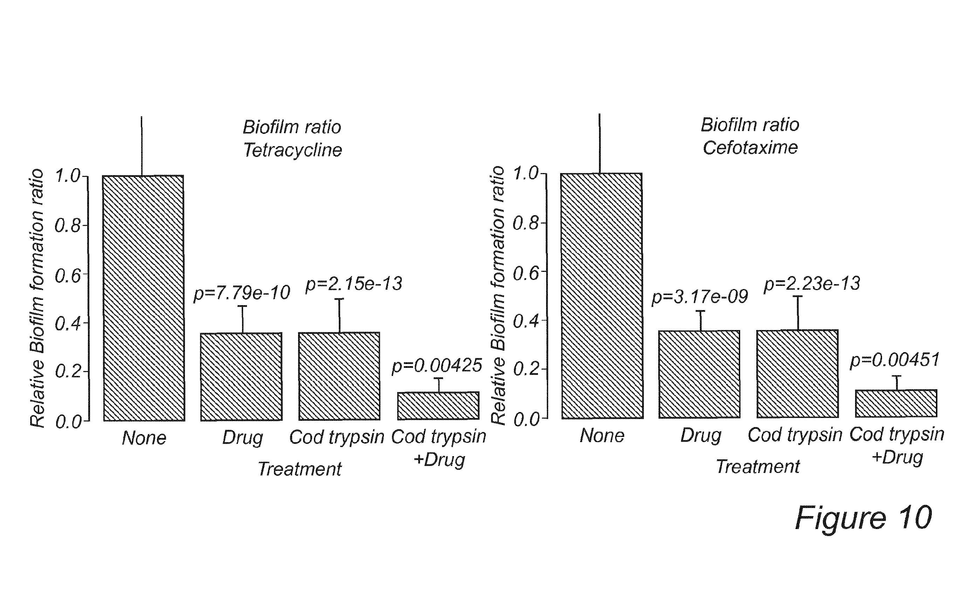

Figure 10. Effects of combination of cod trypsins with selected antibiotics on biofilm dispersal. The figure shows that the combination of cod trypsins with tetracyclin or cefotaxime can be more effective at disrupting biofilms than either alone. Biofilms using a combination of *Streptococcus pneumonia* and *Streptococcus mitis* were grown in a microtiter plate. The biofilm was treated with cod trypsins, an antibiotic, or a combination of the two. The biofilms were stained with crystal violet followed by dissolution with acetic acid. Biofilm formation was measured as absorbance at 492nm, and normalized to untreated biofilm. Based on the study it can be concluded that cod trypsins and antibiotic combinations are more effective in disrupting bacterial biofilm than either cod trypsins or antibiotics alone. P values above bars indicate significance of effects as evaluated by ANOVA analysis.

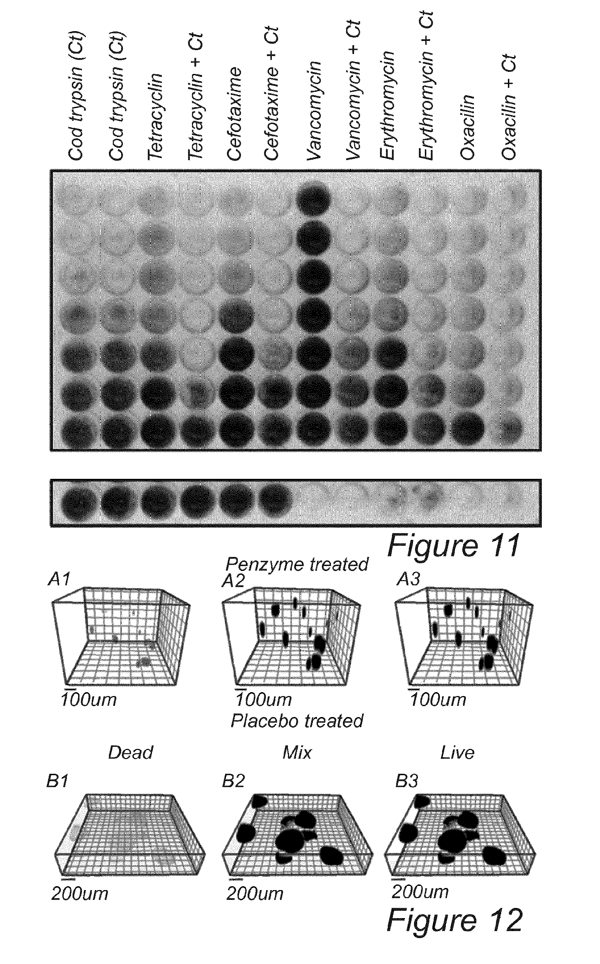

Figure 11. Effects of combination of cod trypsins with selected antibiotics on biofilm dispersal. The image is a clear visual demonstration that the combination of cod trypsins and selected antibiotics can be more effective at disrupting biofilms than either alone. Biofilms using a combination of *Streptococcus pneumonia* and *Streptococcus mitis* were grown in a microtiter plate. The biofilm was treated with cod trypsins, an antibiotic, or a combination of the two. The biofilms were then stained with crystal violet. Based on the study it can be concluded that cod trypsins and antibiotic combinations are more effective in disrupting bacterial biofilm than antibiotics alone.
Figure 12. Treatment of *Pseudomonas aeruginosa* in semi solid gels with cod trypsins. GFP tagged *Pseudomonas aeruginosa* were grown between two layers of 0.5% agar, and placed on a glass slide that fits under the microscope. The bacteria were then treated with cod trypsins, by application of cod trypsin gel on top of the semi solid gels, before being allowed to grow and then compared with untreated biofilms. The biofilm growth was imaged by confocal laser scanning microscopy. The image clearly shows that the cod trypsins limited the growth of the *Pseudomonas* biofilm and caused cell death. By comparing the size of the biofilm it is clear that cod trypsins limited the growth to below a diameter of 100 µm compared to the untreated biofilms being larger than 400 µm.

By the phrase "medical device" is meant, but not limited to, any instrument, apparatus, appliance, software, material or other article for the purpose of diagnosis, prevention, monitoring, treatment, or alleviation of disease, such as diagnosis, monitoring, treatment, alleviation of or compensation; for an injury or handicap, such as investigation, replacement or modification of the anatomy or of a physiological process; control of conception; including devices that do not achieve their principal intended action in or on the human body by pharmacological, immunological or metabolic means - but may be assisted in their function by such means.

The compositions of the present invention, may also optionally include any components in such amounts that the effect of the present invention is not impaired. Examples of the other components conventionally used for cosmetic, medical device and pharmaceutical formulations are liposomes and nanoparticles enabling lipophilic components to be included in the cosmetic-, medical device and pharmaceutical formulations that would not affect the activity of the marine serine proteases such as trypsin. Also, included in the cosmetic, medical device and pharmaceutical formulations may be ultraviolet absorbants (e.g., N,N-dimethyl PABA octyl ester, octyl methyl cinnamate, butyl methoxydibenzoylmethane, di-p-methoxycinnamic acid-mono-2-ehyl hexanoic acid glyceryl, 2-hydroxy-4-methoxy benzophenone, 2-hydroxy-4-methoxy benzophenone-5-sodium sulfonate), lower alcohols (e.g., ethyl alcohol, isopropyl alcohol), preservatives (e.g., methyl paraben, ethyl paraben, propyl paraben, butyl paraben, phenoxy ethanol), bactericides (e.g., chlorohexidine, hydrochloride, trichlorocarbanilide, triclosan, zinc pyrithione), silver (e.g., elemental silver, silver oxide, silver nitrate, silver sulfadiazine, silver nanoparticles), coloring agents (e.g., dyes, pigments), flavoring agents (e.g. menthol, camphor, thymol, eucalyptol) powders, perfumes (e.g., essential oils, perfume of animal origin, synthetic pertume), vitamins (e.g., vitamin A and its derivatives, vitamin E and its derivatives, vitamin C and its derivatives, pantothenic acid, vitamin H, vitamin B and its derivatives), urea, water-soluble polymers (e.g., poly vinyl alcohol, polyvinyl pyrrolidone, carboxyl vinyl polymer, xanthan gum, hyaluronic acid), buffer agents (e.g., sodium glutamate, arginine, aspartic acid, citric acid, sodium citrate, lactic acid, sodium lactate), antibiotic, antifungal, antiviral and antiparasitic drugs.

In one aspect of the invention, there is provided marine serine protease, for use in removal, prevention and inhibition of formation or growth of biofilms. Typically, said marine serine protease is selected from the group of trypsins and chymotrypsins, and any mixture thereof, obtainable from fish, such as cod, including but not limited to, Atlantic cod. Marine serine proteases include, but are not limited to, trypsins, chymotrypsins and elastases. For the purpose of this invention, trypsins and chymotrypsins are preferred.

In one embodiment of this aspect, said marine serine protease is selected from the group of trypsins and chymotrypsins, and any mixture thereof, obtainable from Atlantic cod.

In one embodiment of this aspect, said marine serine protease is trypsin obtainable from Atlantic cod.

In one aspect of the invention, there is provided a composition comprising marine serine protease, for use in removal, prevention and inhibition of formation and growth of biofilms. Typically, said marine serine protease is obtainable from fish, such as cod, including but not limited to, Atlantic cod. Marine serine proteases include, but are not limited to, trypsins and chymotrypsins.

In one embodiment of this aspect, said marine serine protease is selected from the group of trypsins and chymotrypsins, and any mixture thereof obtainable from Atlantic cod.

In one embodiment of this aspect, said marine serine protease is trypsin obtainable from Atlantic cod.

In one embodiment of this aspect, said composition further comprises an antibiotic. Typically, the antibiotic and marine serine protease is present together in one composition. However, the antibiotic and marine serine protease may be used separately or sequentially. Preferably, the marine serine protease is provided first, and thereafter, the antibiotic is provided, since the serine protease would disrupt the biofilm providing an antibiotics improved access to the bacteria, making the effectiveness of the antibiotic greater, in a synergistic manner.

In one embodiment of this aspect, said use further comprises use of an antibiotic.Typically, said antibiotic is used in parallel or sequentially with said composition.

In one embodiment of this aspect, said composition or use further comprises an antibiotic, said antibiotic independently being selected from aminoglycosides, amoxicillin, ampicillin, azithromycin, carbapenems, cefotaxime, ceftriaxone, cefuroxime, cephalosporins, chloramphenicol, ciprofloxacin, clindamycin, dalacin, dalfopristin, daptomycin, doxycycline, ertapenem, erythromycin, fluoroquinolones, meropenem, metronidazole, minocycline, moxifloxacin, nafcillin, oxacillin, penicillin, quinupristin, rifampin, sulfamethoxazole, teicoplanin, tetracycline, trimethoprim, vancomycin, bacitracin and polymyxin B, or a mixture thereof.

In one embodiment of this aspect, said biofilm comprises bacteria independently selected from *Streptococcus pneumonia, Streptococcus mitis, Pseudomonas aeruginosa* and *Heamophilus influenza,* methicillin-resistant *Staphylococcus aureus,* methicillin-susceptible *Staphylococcus aureus, Streptococcus pyogenes, Streptococcus mutans, Streptococcus sanguinis, Legionella pneumophila, Clostridium difficile,* or a mixture thereof.

In one embodiment of this aspect, said biofilm comprises bacteria independently selected from *Streptococcus pneumonia, Streptococcus mitis, Pseudomonas aeruginosa* and *Heamophilus influenza,* or a mixture thereof.

In one embodiment of this aspect, the concentration of trypsin obtainable from cod is 32 U/g or lower, such as 30, 25, 20, 15, 10, 5, 1, 0.1, 0.01 or 0.001 U/g.

In one aspect of the invention, there is provided a method for removal, prevention or inhibition of formation or growth of biofilms, comprising providing a composition comprising marine serine protease. Typically, said marine serine protease is obtainable from fish, such as cod, including but not limited to, Atlantic cod. Marine serine proteases include, but are not limited to, trypsins and chymotrypsins.

In one embodiment of this aspect, said marine serine protease is selected from the group of trypsins and chymotrypsins, and any mixture thereof, obtainable from Atlantic cod.

In one embodiment of this aspect, said marine serine protease is trypsin obtainable from Atlantic cod.

In one embodiment of this aspect, said composition further comprises an antibiotic.

In one embodiment of this aspect, said method further comprises use of an antibiotic.

In one embodiment of this aspect, said method further comprises use of an antibiotic, which is used in parallel or sequentially with said composition.

In one embodiment of this aspect, said composition or method further comprises an antibiotic, said antibiotic independently being selected from aminoglycosides, amoxicillin, ampicillin, azithromycin, carbapenems, cefotaxime, ceftriaxone, cefuroxime, cephalosporins, chloramphenicol, ciprofloxacin, clindamycin, dalacin, dalfopristin, daptomycin, doxycycline, ertapenem, erythromycin, fluoroquinolones, meropenem, metronidazole, minocycline, moxifloxacin, nafcillin, oxacillin, penicillin, quinupristin, rifampin, sulfamethoxazole, teicoplanin, tetracycline, trimethoprim, vancomycin, bacitracin and polymyxin B, or a mixture thereof.

In one embodiment of this aspect, said biofilm comprises bacteria independently selected from *Streptococcus pneumonia, Streptococcus mitis, Pseudomonas aeruginosa* and *Heamophilus influenza,* methicillin-resistant *Staphylococcus aureus,* methicillin-susceptible *Staphylococcus aureus, Streptococcus pyogenes, Streptococcus mutans, Streptococcus sanguinis, Legionella pneumophila, Clostridium difficile,* or a mixture thereof.

In one embodiment of this aspect, said biofilm comprises bacteria independently selected from *Streptococcus pneumonia, Streptococcus mitis, Pseudomonas aeruginosa* and *Heamophilus influenza,* or a mixture thereof.

In one embodiment of this aspect, the concentration of trypsin obtainable from cod is 32 U/g or lower, such as 30, 25, 20, 15, 10, 5, 1, 0.1, 0.01 or 0.001 U/g.

### Examples

The present invention will now be further illustrated in detail but is by no means limited to, the following Examples. Methods for extraction and purification of trypsin from fish, in particular from Atlantic cod, is available in the art, e.g. in WO0078332, including compositions comprising cod trypsins.

### Example 1.

### Inhibition of adhesion of Pneumococcus and S.mitis by a cod trypsin composition

Biofilms using a combination *of Streptococcus pneumonia* and *Streptococcus mitis* were grown in a microtiter plate after being treated with cod trypsins or placebo. After allowing the biofilms to grow for 4 hours the wells were stained with crystal violet followed by dissolution with acetic acid. Biofilm formation was measured as absorbance at 492nm, and normalized to untreated biofilm. Based on the study it is concluded that the cod trypsin composition was very effective in preventing bacterial biofilm formation. Treatment of bacteria with cod trypsin composition prior to biofilm formation showed a concentration dependent effect on biofilm formation *in vitro.* Cod trypsins prevents biofilm formation in a concentration dependent manner. Biofilms using a combination of *Streptococcus pneumonia* and *Streptococcus mitis* were grown in a microtiter plate after being treated with cod trypsins or placebo. After allowing the biofilms to grow for 4 hours the wells were stained with crystal violet followed by dissolution with acetic acid. Biofilm formation was measured as absorbance at 492nm, and normalized to untreated biofilm. Based on the study it can be concluded that cod trypsins are very effective in preventing bacterial biofilm formation. Data is presented as a boxplot where the top of the rectangle indicates the third quartile, a horizontal line near the middle of the rectangle indicates the median, and the bottom of the rectangle indicates the first quartile. A vertical line extends from the top of the rectangle to indicate the maximum value, and another vertical line extends from the bottom of the rectangle to indicate the minimum value. Data for placebo and cod trypsin treated bacteria are placed on the same graph. Overlap of the boxes would indicate no difference in biofilm formation. Statistical significance of the difference, as evaluated by Student's t test, is indicated by symbols above the boxes where n.s. is p > 0.05, * is p < 0.05, ** p < 0.01, and *** p < 0.001. Results are presented in Figure 2. Figure 3 shows the effects of using cod trypsins to treat bacteria before the growing of biofilms.

### Example 2.

### In vitro analysis of disruption of pneumococcus, NTHi and S.mitis biofilms by a cod trypsin composition

A method for biofilm growth of *Pneumococcus* and NTHi was carried out as follows: Bacterial cultures were grown on petri dishes overnight and then suspended in growth medium to an optical density of 0.55 at 620 nm. The suspension was then diluted 1:100 and 200 µl of bacterial solution pipetted into a 96 well plate and grown for 4 hours for *Pneumococcus* or 24 hours for NTHi. Time of growth was selected based on growth curves. After biofilm growth, the broth was removed and each well washed once with phosphate buffered saline (PBS) before inoculation of the cod trypsin composition onto established biofilms in the wells, fresh broth was used as a replacement solution for control wells. After treatment the PBS or cod trypsin solutions were discarded and the remaining biofilm stained with a 1% crystal violet solution, followed by washing. Finally the crystal violet stained biofilm was dissolved using 30% acetic acid and the absorption at 492 nm measured.

### Super Biofilm model

Due to the structural weakness of pneumococcal and non-typable *Haemophilus influenzae* single species biofilms, a structurally stronger biofilm model was developed that composed *Streptococcus pneumonia* and two strains of *Streptococcus mitis.* This model showed high integrity against disruption by saline unlike the single species models that were easily disrupted by incubation with PBS for 5 minutes, see Figure 8.

Bacteria were grown on agar-plates and separated by culture isolation techniques by the different morphology of the bacterial colonies. Three different morphologies were found in the sample, species A, B, and C.

### Characterization of bacterial species within the super biofilm by 16s rRNA sequencing

16S rRNA sequencing of the three bacterial species within the super biofilm species revealed that species A was *Streptococcus pneumonia 70585,* species B was *Streptococcus mitis,* strain B6 and species C was *Streptococcus mitis* strain B6. Difference in 16S rRNA for species B and C were not found but the two strains have different colony morphologies. See Table 1.

**Table 1. 16S rRNA sequencing of biofilm species**

| | | | **Subject** | | | | | **Score** | | | **Identities** | | | |
|---|---|---|---|---|---|---|---|---|---|---|---|---|---|---|
| **#** | **Start** | **End** | | **AC** | **Length** | **Start** | **End** | **Blt** | **Raw** | **EV** | **Match** | **Total** | **%** | **Strand** |
| A | 1 | 1476 | A* | CP00 0918.1 | 2184682 | 16795 | 18261 | 2639 | 1429 | 0 | 1463 | 1477 | 99 | Plus/ Plus |
| B | 1 | 1493 | B* | FN56 8063.1 | 2146611 | 173672 | 175161 | 2704 | 1464 | 0 | 1464 | 1485 | 99 | Plus/ Plus |
| C | 1 | 1476 | C* | FN56 8063.1 | 2146611 | 173687 | 175158 | 2695 | 1459 | 0 | 1471 | 1476 | 99 | Plus/ Plus |

| | | | | | | | | | | | | | | |
|---|---|---|---|---|---|---|---|---|---|---|---|---|---|---|
| A* *Streptococcus pneumoniae* 70585, complete gene B* *Streptococcus mitis* B6 complete gene strain B6 C* *Streptococcus mitis* B6 complete gene strain B6 | | | | | | | | | | | | | | |

Antibiotic susceptibility of the three bacterial species of the super biofilm for selected antibiotics was determined by the zone diameter. Susceptibility was determined according to the European Committee on Antimicrobial Susceptibility Testing Breakpoint tables for interpretation of MICs and zone diameters. S = Susceptible, R = Resistant. Results are presented in Table 2. The table lists the antibiotic susceptibility of the three bacterial species of the super biofilm for selected antibiotics as determined by the zone diameter. Susceptibility was determined according to the European Committee on Antimicrobial Susceptibility Testing Breakpoint tables for interpretation of MICs and zone diameters. S = Susceptible, R = Resistant.

**Table 2. Antibiotic susceptibility of the three super-biofilm species**

| *Drug* | *S. Pneumonia* (A) | | *S*. *mitis* (B) | | *S*. *mitis* (C) | |
|---|---|---|---|---|---|---|
| Tetracycline | 27mm | S | 30mm | S | 29mm | S |
| Vancomycin | 23mm | S | 21mm | S | 21mm | S |
| Oxacillin | 18mm | R | 19mm | R | 20mm | R |
| Cefotaxime | 30mm | S | 31mm | S | 32mm | S |
| Erythromycin | 30mm | S | 38mm | S | 35mm | S |
| Dalacin | 27mm | S | 33mm | S | 31mm | S |
| Chloramphenicol | 27mm | S | 31mm | S | 29mm | S |
| Trimethoprim | 6mm | R | 28mm | S | 25mm | S |
| Ceftriaxone | 32mm | S | 37mm | S | 37mm | S |
| Ampicillin | 37mm | S | 34mm | S | 33mm | S |
| Cefuroxime | 32mm | S | 41mm | S | 37mm | S |
| Ciprofloxacin | 24mm | S | 15mm | R | 18mm | S |
| Penicillin | 26mm | S | 25mm | S | 27mm | S |
| Ertapenem | 34mm | S | 32mm | S | 32mm | S |
| Meropenem | 38mm | S | 37mm | S | 36mm | S |

### Cod trypsins treatment of Super biofilm

The super biofilm with a combination of *Streptococcus pneumonia* and *Streptococcus mitis* were grown in a microtiter plate. The biofilm was treated with cod trypsins or placebo for 2 minutes and the wells were stained to measure the presence (black wells) of biofilm or its absence (clear wells) after treatment (see Figure 4). Based on the study it can be concluded that cod trypsins is very effective in disrupting the super-biofilm.Treatment of the super biofilm model with cod trypsins after biofilm formation and growth showed that cod trypsins disrupted biofilms in a concentration dependent manner. Data in Figure 5 are presented as a boxplot where the top of the rectangle indicates the third quartile, a horizontal line near the middle of the rectangle indicates the median, and the bottom of the rectangle indicates the first quartile. A vertical line extends from the top of the rectangle to indicate the maximum value, and another vertical line extends from the bottom of the rectangle to indicate the minimum value. Data for placebo and cod trypsin treated bacterial biofilms are placed on the same graph. Overlap of the boxes indicate no difference in biofilm formation, while greater distance between the boxes in the same column indicates a greater difference as represented by the Log reduction of biofilm formation. In the tests presented in Figure 6, the biofilm was treated with cod trypsins or placebo and the wells were stained with crystal violet followed by dissolution with acetic acid. Biofilm formation was measured as absorbance at 492nm, and the reduction compared to placebo treated biofilm on a logarithmic scale. Based on the study it can be concluded that cod trypsins is very effective in disrupting bacterial biofilm at concentrations 4 U/g or higher, in less than 2 minutes, where a log reduction of 3 represent 99.9% removal of the biofilm.

Testing combination biofilms comprised of *S.pneumonia* and *Heamophilus influenza* species showed that cod trypsins were effective in eradicating biofilms of these species as well as the super biofilm. Figure 7 shows that biofilms formed by the super biofilm species *(S. pneumonia* and the two *S.mitis* strains, columns 5 and 6) were far stronger than *S.pneumonia* alone (columns 1 and 2) or *S.pneumonia* in combination with *H.influenza* (column 3 and 4). The super biofilm was also more tolerant to PBS (white columns) and low concentration of cod trypsins (black columns) washing. Biofilms using a combination of *Streptococcus pneumonia* and *Streptococcus mitis* or *Haemophilus influenza* were grown in a microtiter plate. The biofilm was washed briefly with cod trypsins or PBS and the wells were stained with crystal violet followed by dissolution with acetic acid. Biofilm formation was measured as absorbance at 492nm, and normalized to untreated biofilm. Figure 8 shows that cod trypsins (white columns) were effective in disrupting biofilms formed by combinations of three strains of *S*. *pneumonia* and two strains of *H. influenza.* Strains 1, 2, and 3 are *S*. *pneumonia* species, and strains 7 and 8 are *H. influenza species.* The graph shows that cod trypsins were effective at degrading biofilms of all combinations of all bacterial strains. Biofilms using a combination of *Streptococcus pneumonia* and/or *Haemophilus influenza* were grown in a microtiter plate. The biofilm was treated with cod trypsins or PBS and the wells were stained with crystal violet followed by dissolution with acetic acid. Biofilm formation was measured as absorbance at 492nm. It is clear from the figure that single species biofilms are weak and can easily be disrupted by washing with PBS. On the other hand the stronger biofilms formed by two or more bacterial species required cod trypsin treatment for disruption.

### Example 3.

### Combination of cod trypsin and antibiotics for superior biofilm disruption by such trypsin compositions

Biofilms using a combination of *Streptococcus pneumonia* and *Streptococcus mitis* were grown in a microtiter plate. The biofilm was treated with an antibiotic or a combination of the antibiotics and cod trypsins. The biofilms were stained with crystal violet followed by dissolution with acetic acid. Biofilm formation was measured as absorbance at 492nm, and normalized to untreated biofilm. It was concluded that cod trypsins and antibiotic combinations were more effective in disrupting bacterial biofilm than antibiotics alone (see Figure 9). P values above bars indicate significance of difference between the two treatments as evaluated by Student's t test. Using selected antibiotics in combination with cod trypsins treatment showed that the use of cod trypsins significantly enhanced the effects of the antibiotics in 4 out of the 5 drugs tested. The data in Figure 10 provide results where the biofilms were stained with crystal violet followed by dissolution with acetic acid. Biofilm formation was measured as absorbance at 492nm, and normalized to untreated biofilm. P values above bars indicate significance of effects as evaluated by ANOVA analysis.The data demonstrate that the local combinatory use of cod trypsins with antibiotic treatment improved the disruption of biofilm compared to antibiotics alone. It was surpisingly identified that a combination of cod trypsins with antibiotics improved the biofilm disruption by antibiotics that allow the use of lower concentrations of antibiotics when treating biofilm infections. Figure 11 shows effects of combination of cod trypsins with selected antibiotics on biofilm dispersal. The image is a clear visual demonstration that the combination of cod trypsins and selected antibiotics was more effective at disrupting biofilms than either alone. Biofilms using a combination of *Streptococcus pneumonia* and *Streptococcus mitis* were grown in a microtiter plate. The biofilm was treated with cod trypsins, an antibiotic, or a combination of the two. The biofilms were then stained with crystal violet. Based on the study it can be concluded that cod trypsins and antibiotic combinations are more effective in disrupting bacterial biofilm than antibiotics alone.

### Example 4.

### Treatment of Pseudomonas aeruginosa in semi solid gels with trypsin compositions

GFP tagged *Pseudomonas aeruginosa* were grown between two layers of 0.5% agar, and placed on a glass slide that fits under the microscope. The bacteria were then treated with cod trypsins before being allowed to grow and then compared to untreated biofilms. The biofilm growth was imaged by confocal laser scanning microscopy. The results showed that cod trypsins limit the growth of the *Pseudomonas* biofilm and causes cell death. By comparing the size of the biofilm it is clear that cod trypsins limited the growth to below a diameter of 100 µm compared to the untreated biofilms being larger than 400 µm.

### Items of the invention

1. Marine serine protease, for use in removal, prevention and inhibition of formation or growth of biofilms.
2. The marine serine protease for use of item 1, wherein said marine serine protease is selected from the group of trypsins and chymotrypsins, and any mixture thereof, obtainable from Atlantic cod.
3. A composition comprising marine serine protease, for use in removal, prevention and inhibition of formation and growth of biofilms.
4. The composition for use of item 3, wherein said marine serine protease is selected from the group of trypsins and chymotrypsins, and any mixture thereof, obtainable from Atlantic cod.
5. The composition for use of item 3 or 4, wherein said marine serine protease is trypsin obtainable from Atlantic cod.
6. The composition for use of any one of items 3 to 5, wherein said composition further comprises an antibiotic.
7. The composition for use of any one of items 3 to 5, wherein said use further comprises use of an antibiotic.
8. The composition for use of item 7, wherein said use further comprises use of an antibiotic, which is used in parallel or sequentially with said composition.
9. The composition for use of any one of items 3 to 8, wherein said composition or use further comprises an antibiotic, said antibiotic independently being selected from aminoglycosides, amoxicillin, ampicillin, azithromycin, carbapenems, cefotaxime, ceftriaxone, cefuroxime, cephalosporins, chloramphenicol, ciprofloxacin, clindamycin, dalacin, dalfopristin, daptomycin, doxycycline, ertapenem, erythromycin, fluoroquinolones, meropenem, metronidazole, minocycline, moxifloxacin, nafcillin, oxacillin, penicillin, quinupristin, rifampin, sulfamethoxazole, teicoplanin, tetracycline, trimethoprim, vancomycin, bacitracin and polymyxin B, or a mixture thereof.
10. The composition for use, according to any one of items 3 to 9, wherein said biofilm comprises bacteria independently selected from *Streptococcus pneumonia, Streptococcus mitis, Pseudomonas aeruginosa* and *Heamophilus influenza,* methicillin-resistant *Staphylococcus aureus,* methicillin-susceptible *Staphylococcus aureus, Streptococcus pyogenes, Streptococcus mutans, Streptococcus sanguinis, Legionella pneumophila, Clostridium difficile,* or a mixture thereof.
11. The composition for use, according to any one of items 3 to 9, wherein said biofilm comprises bacteria independently selected from *Streptococcus pneumonia, Streptococcus mitis, Pseudomonas aeruginosa* and *Heamophilus influenza,* or a mixture thereof.
12. The composition for use, according to any one of items 4 to 11, wherein the concentration of trypsin obtainable from cod is 32 U/g or lower.
13. Method for removal, prevention or inhibition of formation or growth of biofilms, comprising providing a composition comprising marine serine protease.
14. The method of item 13, wherein said marine serine protease is selected from the group of trypsins and chymotrypsins, and any mixture thereof, obtainable from Atlantic cod.
15. The method of item 13 or 14, wherein said marine serine protease is trypsin obtainable from Atlantic cod.
16. The method of any one of items 13 to 15, wherein said composition further comprises an antibiotic.
17. The method of any one of items 13 to 15, wherein said method further comprises use of an antibiotic.
18. The method of item 17, wherein said method further comprises use of an antibiotic, which is used in parallel or sequentially with said composition.
19. The method of any one of items 13 to 18, wherein said composition or method further comprises an antibiotic, said antibiotic independently being selected from aminoglycosides, amoxicillin, ampicillin, azithromycin, carbapenems, cefotaxime, ceftriaxone, cefuroxime, cephalosporins, chloramphenicol, ciprofloxacin, clindamycin, dalacin, dalfopristin, daptomycin, doxycycline, ertapenem, erythromycin, fluoroquinolones, meropenem, metronidazole, minocycline, moxifloxacin, nafcillin, oxacillin, penicillin, quinupristin, rifampin, sulfamethoxazole, teicoplanin, tetracycline, trimethoprim, vancomycin, bacitracin and polymyxin B, or a mixture thereof.
20. The method of any one of items 13 to 19, wherein said biofilm comprises bacteria independently selected from *Streptococcus pneumonia, Streptococcus mitis, Pseudomonas aeruginosa* and *Heamophilus influenza,* methicillin-resistant *Staphylococcus aureus,* methicillin-susceptible *Staphylococcus aureus, Streptococcus pyogenes, Streptococcus mutans, Streptococcus sanguinis, Legionella pneumophila, Clostridium difficile,* or a mixture thereof.
21. The method of any one of items 13 to 19, wherein said biofilm comprises bacteria independently selected from *Streptococcus pneumonia, Streptococcus mitis, Pseudomonas aeruginosa* and *Heamophilus influenza,* or a mixture thereof.
22. The method of any one of items 13 to 21, wherein the concentration of trypsin obtainable from cod is 32 U/g or lower.

## Claims

1. Marine serine protease, for use in removal, prevention and inhibition of formation or growth of biofilms.

2. The marine serine protease for use of claim 1, wherein said marine serine protease is selected from the group of trypsins and chymotrypsins, and any mixture thereof, obtainable from Atlantic cod.

3. A composition comprising marine serine protease, for use in removal, prevention and inhibition of formation and growth of biofilms.

4. The composition for use of claim 3, wherein said marine serine protease is selected from the group of trypsins and chymotrypsins, and any mixture thereof, obtainable from Atlantic cod.

5. The composition for use of claim 3 or 4, wherein said marine serine protease is trypsin, obtainable from Atlantic cod

6. The composition for use of any one of claims 3 to 5, wherein said composition further comprises an antibiotic.

7. The composition for use of any one of claims 3 to 5, wherein said use further comprises use of an antibiotic.

8. The composition for use of claim 7, wherein said use further comprises use of an antibiotic, which is used in parallel or sequentially with said composition.

9. The composition for use of any one of claims 3 to 8, wherein said composition or use further comprises an antibiotic, said antibiotic independently being selected from aminoglycosides, amoxicillin, ampicillin, azithromycin, carbapenems, cefotaxime, ceftriaxone, cefuroxime, cephalosporins, chloramphenicol, ciprofloxacin, clindamycin, dalacin, dalfopristin, daptomycin, doxycycline, ertapenem, erythromycin, fluoroquinolones, meropenem, metronidazole, minocycline, moxifloxacin, nafcillin, oxacillin, penicillin, quinupristin, rifampin, sulfamethoxazole, teicoplanin, tetracycline, trimethoprim, vancomycin, bacitracin and polymyxin B, or a mixture thereof.

10. The composition for use, according to any one of claims 3 to 9, wherein said biofilm comprises bacteria independently selected from *Streptococcus pneumonia, Streptococcus mitis, Pseudomonas aeruginosa* and *Heamophilus influenza,* methicillin-resistant *Staphylococcus aureus,* methicillin-susceptible *Staphylococcus aureus, Streptococcus pyogenes, Streptococcus mutans, Streptococcus sanguinis, Legionella pneumophila, Clostridium difficile,* or a mixture thereof.

11. The composition for use, according to any one of claims 3 to 9, wherein said biofilm comprises bacteria independently selected from *Streptococcus pneumonia, Streptococcus mitis, Pseudomonas aeruginosa* and *Heamophilus influenza,* or a mixture thereof.

12. The composition for use, according to any one of claims 4 to 11, wherein the concentration of trypsin obtainable from cod is 32 U/g or lower.
